(19) Europäisches Patentamt
European Patent Office
Office européen des brevets

(11) EP 4 282 976 B1

(12) **EUROPEAN PATENT SPECIFICATION**

(45) Date of publication and mention of the grant of the patent:
**09.04.2025 Bulletin 2025/15**

(21) Application number: **22209879.0**

(22) Date of filing: **28.11.2022**

(51) International Patent Classification (IPC):
***C12Q 1/25*** (2006.01) ***G01N 33/68*** (2006.01)

(52) Cooperative Patent Classification (CPC):
**G01N 33/6812; C12Q 1/25; G01N 33/6839**

(54) **COLORIMETRIC SENSOR FOR DETECTION OF L-ASPARAGINE, PRODUCTION METHOD AND APPLICATIONS THEREOF**

KOLORIMETRISCHER SENSOR ZUM NACHWEIS VON L-ASPARAGIN, HERSTELLUNGSVERFAHREN UND ANWENDUNGEN DAVON

CAPTEUR COLORIMÉTRIQUE POUR LA DÉTECTION DE L-ASPARAGINE, PROCÉDÉ DE PRODUCTION ET APPLICATIONS DE CELUI-CI

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **27.05.2022 PT 2022118012**
**31.05.2022 EP 22176588**
**25.11.2022 PT 2022118358**

(43) Date of publication of application:
**29.11.2023 Bulletin 2023/48**

(73) Proprietor: **Universidade de Aveiro**
**3810-193 Aveiro (PT)**

(72) Inventors:
- **FREIRE MARTINS, MARA GUADALUPE**
  **3810-193 AVEIRO (PT)**
- **CARVALHO NEVES, MÁRCIA**
  **3830-203 ÍLHAVO (PT)**
- **MORA TAVARES, ANA PAULA**
  **3850-581 BRANCA (PT)**
- **FONSECA NUNES, JOÃO CLÁUDIO**
  **3810-193 AVEIRO (PT)**

(74) Representative: **Patentree**
**Edificio Net**
**Rua de Salazares, 842**
**4149-002 Porto (PT)**

(56) References cited:
- **KUMAR KULDEEP ET AL: "Plant asparaginase-based asparagine biosensor for leukemia", ARTIFICIAL CELLS, NANOMEDICINE AND BIOTECHNOLOGY, vol. 41, no. 3, 19 September 2012 (2012-09-19), US, pages 184 - 188, XP093090964, ISSN: 2169-1401, DOI: 10.3109/10731199.2012.716062**
- **AGGARWAL SHALU ET AL: "A comprehensive review on incredible renewable carriers as promising platforms for enzyme immobilization & thereof strategies", INTERNATIONAL JOURNAL OF BIOLOGICAL MACROMOLECULES, ELSEVIER BV, NL, vol. 167, 10 November 2020 (2020-11-10), pages 962 - 986, XP086440272, ISSN: 0141-8130, [retrieved on 20201110], DOI: 10.1016/J.IJBIOMAC.2020.11.052**
- **NUNES JOÃO C. F. ET AL: "Enhanced Enzyme Reuse through the Bioconjugation of L-Asparaginase and Silica-Based Supported Ionic Liquid-like Phase Materials", MOLECULES, vol. 27, no. 3, 29 January 2022 (2022-01-29), pages 929, XP093090994, DOI: 10.3390/molecules27030929**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

The present disclosure relates to a portable colorimetric enzymatic biosensor for the visual detection of the concentration levels of the amino acid L-asparagine in aqueous solutions.

### BACKGROUND

The document "Nunes, J.C.F.; Cristóvão, R.O.; Santos-Ebinuma, V.C.; Faria, J.L.; Silva, C.G.; Neves, M.C.; Freire, M.G.; Tavares, A.P.M. L-Asparaginase-based biosensors. Encyclopedia. 2021, 1, 848-858" is a Review article summarizing different L-Asparaginase-based biosensors for the quantification of L-asparagine. The reported articles in the review article use as detection methodologies the following techniques or reagents: Potentiometric ammonia gas detector, Ammonia gas electrode, Polypyrrole probe, Ammonium-selective glass electrode and the reagents: Nessler reagent (read in a spectrophotometer) and Phenol red (pH changes). Therefore, this document is silent on portable sensors capable of changing their color in the presence of different concentrations of L-asparagine, without the need of using any further lab equipment, electrodes, detectors or phenol red reagent.

The document "Nunes, J.C.F.; Almeida, M.R.; Bento, R.M.F.; Pereira, M.M.; Santos-Ebinuma, V.C.; Neves, M.C.; Freire, M.G.; Tavares, A.P.M. Enhanced enzyme reuse through the bioconjugation of L-asparaginase and silica-based supported ionic liquid-like phase materials. Molecules. 2022, 27, 1-21." describes the use of modified solid silica gel materials (Commercial silica gel 60 (0.2-0.5 mm), spherical particles) with ionic liquids for the immobilization of the enzyme L-asparaginase and its quantification through biological activity tests with Nessler reagent (output read in a spectrophotometer). Regardless, this document does not describe a visual biosensor based on modified silica fabric with ionic liquids for the visual quantification of the amino acid L-asparagine in aqueous solutions, wherein the physical support for the enzyme changes its color depending on the L-asparagine concentration range.

The document "Kotzia, G.A.; Labrou, N.E. Engineering substrate specificity of E. carotovora L-asparaginase for the development of biosensor. J. Mol. Catal. B Enzym. 2011, 72, 95-101" describes the immobilization of Leu71lle mutant L-asparaginase by crosslinking with glutaraldehyde on the side of a transparent plastic cuvette for the quantification of L-asparagine using the Nessler reagent (output read in a spectrophotometer). This document thus discloses a technology that requires a spectrophotometer to quantify the presence of L-asparagine.

The document "Labrou, N.E.; Muharram, M.M. Biochemical characterization and immobilization of Erwinia carotovora L-asparaginase in a microplate for high-throughput biosensing of L-asparagine. Enzym. Microb. Technol. 2016, 92, 86-93" describes an assay for the determination of L-asparagine with a microplate-based biosensor using L-asparaginase immobilized by crosslinking with glutaraldehyde and deposited into the well of a microplate in 96-well format and Nessler reagent (output read in a microplate equipment). Similarly, the detection of L-asparagine disclosed in this document is dependent on laboratory equipment, in this case a microplate reader.

The document US 2010/0190658 A1 describes a colorimetric assay for the determination of organic substituents with primary or secondary amines or with thiol groups immobilized on or in insoluble materials. The visual detection or quantification of the solid-phase bound primary and secondary amines and thiol groups, comprises the addition of a fluid (acetone, acetonitrile, DMF (dimethylformamide), DMSO (dimethylsulfoxide), methanol, water or mixtures) to a substrate (animal or plant tissue, array surfaces, bone, dipsticks, macro-beads, membranes, microplates, nanoparticles, polymer, resin, silica gel, among others), and the further addition of DESC reagent (1-methyl-2-(4'-nitrophenyl)-imidazo[1,2-a] pyrimidinium perchlorate, doi: 10.1021/cc800031y). The color change intensity is proportional to the total amino groups and/or thiol groups concentration, detected via visual observation, optical microscope, or spectroscopically following 1 min - 10 min of DESC reagent addition. The document is however silent on a portable visual biosensor for the rapid detection of L-asparagine levels in aqueous solutions.

The document WO 1994/05805 A1 describes a method and assay kit for the determination of the enzymatic activity of asparagine-β-amido hydrolase enzymes (glycosylasparaginases (EC 3.5.1.26) and asparaginases (EC 3.5.1.1)) in biological samples. In this method it is necessary the contact/incubation of the samples with a compound (that acts as a substrate (L-asparagine, L-aspartic acid derivatives with a free α-amino and α-carboxyl group of known fluorogenic compounds (7-amino-4-methylcoumarin, β-methylumbelliferone, fluorescein, β-naphtylamide, resorufin, 6-aminoquinoline or resorcinol), derivatives of known coloured reagents (β-naphtylamide) or compounds used in chemiluminescence (D-luciferine or adamantyl-1,2-dioxethane) in a suitable buffer solution) containing a luminescent (fluorescent, phosphorescent or chemiluminescent) or a colored moiety attached to the β-amino group of L-asparagine or β-carboxyl group of L-aspartic acid. This label compound is cleaved off via the catalysis of asparagine-β-amido hydrolase enzymes present in the biological samples. The amount of released moiety is determined through luminometry (fluorometry or chemiluminometry) or spectrophotometry, quantitatively measuring the amount of asparagine-β-amido hydrolase enzymes present in the biological samples. The document is however silent on a portable visual biosensor for

the rapid detection of L-asparagine levels in aqueous solutions, which does not require any further lab equipment such as luminometer, fluorometer, chemiluminometer or a spectrophotometer.

The document WO 2008/013530 A1 describes a method for direct detection and quantitation of asparagine synthetase, an enzyme responsible for the cellular asparagine production, whose expression has been correlated with chemoresistance to certain cancer types, in biological samples. The method comprises substantial purification of asparagine synthetase protein from a sample (through SDS-PAGE separation, size exclusion chromatography, cation or anion exchange chromatography or antibody-mediated enrichment), adding standards (stable isotope peptide standards) to the protein and determining the amount of protein in the sample using mass spectrometry. The document is however silent on a portable visual biosensor for the rapid detection of L-asparagine levels in aqueous solutions, that does not require a previous purification of the compound to be detected, i.e., the L-asparagine amino acid.

These facts are disclosed in order to illustrate the technical problem addressed by the present disclosure.

## GENERAL DESCRIPTION

The present disclosure relates to a biosensor based on silica fabric functionalized with ionic liquids for the attachment of the enzyme L-asparaginase and further detection of the L-asparagine amino acid. The functionalization of the silica fabric with ionic liquids enhances the binding/adsorption of the L-asparaginase enzyme (ASNase) through simple experimental procedures, thus allowing the detection of L-asparagine amino acid by visual observation. In an embodiment, upon addition of a solution into the modified fabric, and if L-asparagine is present in said solution, it interacts with the immobilized enzyme L-asparaginase, and is converted in L-aspartic acid and ammonium ions. By adding a revealing agent, such as Nessler's solution that changes color in the presence of ammonium ions, the color of the fabric changes as a consequence of the presence of ammonium ions, and consequently the presence of L-asparagine amino acid. Surprisingly, the color change of the functionalized fabric is proportional to the concentration of L-asparagine. Thus, the disclosed biosensor is a simple device that can be used for rapid detection of L-asparagine levels in aqueous solutions, by simple observation of change of color in a fabric, and without the need of special equipment such as microplate readers or spectrophotometers.

In an embodiment, the disclosed biosensor is a portable biosensor, which changes its color with the changes in the L-asparagine concentration levels. In a further embodiment, the biosensor is a visual biosensor based on modified silica fabric with ionic liquids for the visual quantification of the amino acid L-asparagine in aqueous solutions, wherein the silica fabric changes the color depending on the L-asparagine concentration range. The disclosed biosensor does not require the use of a spectrophotometer, microplate readers, or any lab equipment for the quantification of the L-asparagine levels, allowing its fast and simple determination.

In an embodiment, the visual detection of the L-asparagine consists on the change of the color of the modified silica fabric material where each concentration range of L-asparagine corresponds to a specific color.

In an embodiment, for the modification of the silica fabric, firstly a silane compatibilizing agent/anion source, namely (3-Chloropropyl)trimethoxysilane ($C_6H_{15}ClO_3Si$), is added to the silica fabric. Then, after washing with organic solvents, such as toluene, ethanol, methanol, or mixtures thereof, and water, the cation (N,N-dimethylbutylammonium ($[N_{3114}]^+$) or triethylammonium ($[N_{3222}]^+$) is added and then washed with organic solvents, such as toluene, methanol or mixtures thereof, and water.

In an embodiment, high concentration ranges of L-asparagine ($10^{-1}$ M and $10^{-2}$ M) in aqueous solutions are detected by visual means, due to the change of the color of the modified silica fabrics ($[Si][N_{3114}]Cl$ and $[Si][N_{3222}]Cl$). The visual detection includes a sequential addition of reduced volumes of aqueous solutions of L-asparaginase, sample (containing L-asparagine) and revealing solution (Nessler's reagent). Depending on the L-asparagine concentration levels, different colors of the modified silica fabric are obtained ranging from white-grey to brownish red.

The present disclosure relates to a fabric for immobilization of L-asparaginase enzyme, the fabric comprising: a plurality of silica fibers; and an ionic liquid supported on the silica fibers, wherein the ionic liquid comprises a quaternary ammonium cation and chloride as counterion; wherein the L-asparaginase enzyme is immobilized on the plurality of silica fibers through the ionic liquid.

The invention relates to a fabric for detecting a L-asparagine amino acid in a sample as defined in claim 1, wherein the fabric comprises: a plurality of silica fibers; an ionic liquid linked to the plurality of silica fibers by a spacer arm, wherein the ionic liquid comprises a quaternary ammonium cation and a chloride as counterion; and a L-asparaginase enzyme immobilized in the plurality of silica fibers though the ionic liquid; wherein the spacer arm comprises a compound with formula $C_xH_yClO_3Si$, wherein X is an entire number ranging from 3 to 12 and wherein Y is an entire number ranging from 10 to 30; and wherein the presence of L-asparagine in the sample is detectable by a change of color of the fabric after addition of a revealing solution.

The invention relates to a fabric for detecting a L-asparagine amino acid in a sample as defined in claim 1, wherein the fabric comprises: a plurality of silica fibers; an ionic liquid linked to the plurality of silica fibers by a spacer arm, wherein the ionic liquid comprises a quaternary ammonium cation and a chloride as counterion; and wherein the spacer arm

comprises a compound with formula $C_xH_yClO_3Si$, wherein X is an entire number ranging from 3 to 12 and wherein Y is an entire number ranging from 10 to 30; and wherein the presence of L-asparagine in the sample is detectable by a change of color of the fabric after addition of a L-asparaginase enzyme solution to the fabric, addition of the sample, and addition of a revealing solution.

In the invention, the spacer arm ($C_xH_yClO_3Si$) is selected from a list comprising (3-Chloropropyl)trimethoxysilane ($C_6H_{15}ClO_3Si$), (3-Chloropropyl)triethoxysilane ($C_9H_{21}ClO_3Si$), (Chloromethyl)trimethoxysilane ($C_4H_{11}ClO_3Si$), (Chloromethyl)triethoxysilane ($C_7H_{17}ClO_3Si$), (2-Chloroethyl)trimethoxysilane ($C_5H_{13}ClO_3Si$), (2-Chloroethyl)triethoxysilane ($C_8H_{19}ClO_3Si$), (4-Chlorobutyl)trimethoxysilane ($C_7H_{17}ClO_3Si$), (4-Chlorobutyl)triethoxysilane ($C_{10}H_{23}ClO_3Si$), or combinations thereof; preferably wherein the spacer arm is a (3-Chloropropyl)trimethoxysilane.

In an embodiment, the revealing solution is Nessler's reagent or phenol red, preferably Nessler's reagent.

In an embodiment, the silica fibers are woven.

In an embodiment, the quaternary ammonium cation comprised in the ionic liquid is selected from N,N-dimethylbutylammonium or triethylammonium.

In an embodiment, the mass of ionic liquid per mass of fabric ranges from 8 $mg.g^{-1}$ to 30 $mg.g^{-1}$, preferably from 8 $mg.g^{-1}$ to 25 $mg.g^{-1}$.

In an embodiment, the diameter of the silica fibers ranges from 7 to 11 $\mu$m, preferably from 9 to 11 $\mu$m, measured by scanning electron microscopy.

In an embodiment, the sample is a liquid biological sample, or a liquid food-derived sample. In a further embodiment, the liquid biological sample is a blood serum sample.

In an embodiment, the concentration of L-asparaginase enzyme, preferably the immobilized enzyme, in the fabric ranges from 0.1 $mg/cm^2$ to 0.5 $mg/cm^2$, preferably from 0.2 $mg/cm^2$ to 0.5 $mg/cm^2$.

An aspect of the present disclosure relates to the use of a fabric as defined in claim 1 in a sensor for detecting L-asparagine amino acid in an aqueous solution.

In an embodiment, the aqueous solution is a serum sample, or a food-derived sample.

The present disclosure also relates to a portable sensor for detecting L-asparagine amino acid in an aqueous solution comprising a fabric as defined in claim 1 for detecting a L-asparagine amino acid, preferably a sensor for monitoring and/or diagnosing a disease that causes an increase in the concentration of L-asparagine amino acid in blood, more preferably a sensor for monitoring and/or diagnosing leukemia disease.

In an embodiment, the immobilized L-asparaginase enzyme is available to catalyze the hydrolysis of the L-asparagine amino acid present in the aqueous solution into ammonia and L-aspartic acid. The produced ammonia, resultant from the presence of L-asparagine, is then detectable after addition of the revealing solution.

In an embodiment, the sensor is arranged to detect L-asparagine amino acid in a concentration ranging from $10^{-5}$ M to 10 M, preferably $10^{-3}$ M to $10^{-1}$ M.

An aspect of the present disclosure relates to a method for detecting L-asparagine amino acid in an aqueous solution using the fabric as defined in claim 1 or the portable sensor defined in claim 9, the method comprising the following steps: adding the aqueous solution to the portable sensor or fabric; adding a revealing solution to the portable sensor or fabric, preferably wherein the revealing solution is Nessler's reagent; analyzing the fabric or portable sensor for a change of color, wherein a change of color indicates the presence of L-asparagine amino acid in the aqueous solution.

An aspect of the present disclosure relates to a kit for detecting L-asparagine amino acid comprising the fabric as defined in claim 1 or the disclosed portable sensor as defined in claim 9 and a revealing agent. In a further embodiment, the kit also comprises a color scale to correlate the color of the disclosed fabric with the concentration of L-asparagine amino acid in the aqueous solution.

In an embodiment, the revealing agent is Nessler's reagent.

In an embodiment, the kit is for diagnosing and or monitoring a disease that causes an increase in the concentration of L-asparagine amino acid in blood, such as leukemia disease.

## BRIEF DESCRIPTION OF THE DRAWINGS

The following figures provide preferred embodiments for illustrating the disclosure and should not be seen as limiting the scope of invention.

**Figure 1:** Gradient color scale representation of the RGB color code converted from CIE-L*a*b* color coordinates and corresponding macroscopic aspect of silica fabric regarding aqueous solution of different L-asparagine concentration levels ($10^{-1}$ M, $10^{-2}$ M, $10^{-3}$ M, $10^{-4}$ M and $10^{-5}$ M).

**Figure 2:** Gradient color scale representation of the RGB color code converted from CIE-L*a*b* color coordinates and corresponding macroscopic aspect of [Si][$N_{3114}$]Cl (silica fabric modified with [$N_{3114}$]Cl) regarding aqueous solution of different L-asparagine concentration levels ($10^{-1}$ M, $10^{-2}$ M, $10^{-3}$ M, $10^{-4}$ M and $10^{-5}$ M).

**Figure 3:** Gradient color scale representation of the RGB color code converted from CIE-L*a*b* color coordinates and corresponding macroscopic aspect of [Si][$N_{3222}$]Cl (silica fabric modified with [$N_{3222}$]Cl) regarding aqueous solution of different L-asparagine concentration levels ($10^{-1}$ M, $10^{-2}$ M, $10^{-3}$ M, $10^{-4}$ M and $10^{-5}$ M).

**Figure 4:** Gradient color scale representation of the RGB color code converted from CIE-L*a*b* color coordinates and corresponding macroscopic aspect of [Si][$N_{3114}$]Cl (silica fabric modified with [$N_{3114}$]Cl) regarding commercial blood human serum with different L-asparagine concentration levels ($10^{-1}$ M, $10^{-2}$ M, $10^{-3}$ M, $10^{-4}$ M and $10^{-5}$ M).

**Figure 5:** Gradient color scale representation of the RGB color code converted from CIE-L*a*b* color coordinates and corresponding macroscopic aspect of [Si][$N_{3222}$]Cl (silica fabric modified with [$N_{3222}$]Cl) regarding commercial blood human serum with different L-asparagine concentration levels ($10^{-1}$ M, $10^{-2}$ M, $10^{-3}$ M, $10^{-4}$ M and $10^{-5}$ M).

**Figure 6:** ATR-FTIR spectra of: silica fabric (a), ASNase-silica fabric (b), [Si][$N_{3114}$]Cl (c), ASNase-[Si][$N_{3114}$]Cl (d), [Si][$N_{3222}$]Cl (e), ASNase-[Si][$N_{3222}$]Cl (f). Amide group of the polypeptide chain of ASNase (dotted line): 1700-1600 $cm^{-1}$; siloxane groups (Si-O-Si) of silica (dashed line): 1100-1000 $cm^{-1}$. ([Si][$N_{3114}$]Cl: silica fabric modified with [$N_{3114}$]Cl; [Si][$N_{3222}$]Cl: silica fabric modified with [$N_{3222}$]Cl; ASNase: L-asparaginase).

**Figure 7:** Scanning electron microscopy (SEM) images of silica fabric, silica fabric modified with [$N_{3114}$]Cl ([Si][$N_{3114}$]Cl), and silica fabric modified with [$N_{3222}$]Cl ([Si][$N_{3222}$]Cl).

**Figure 8:** 3D representation of CIE-L*a*b* color coordinates of silica fabric regarding aqueous solution of different L-asparagine concentration levels ($10^{-1}$ M, $10^{-2}$ M, $10^{-3}$ M, $10^{-4}$ M and $10^{-5}$ M).

**Figure 9:** 3D representation of CIE-L*a*b* color coordinates of silica fabric modified with [$N_{3114}$]Cl ([Si][$N_{3114}$]Cl) regarding aqueous solution of different L-asparagine concentration levels ($10^{-1}$ M, $10^{-2}$ M, $10^{-3}$ M, $10^{-4}$ M and $10^{-5}$ M).

**Figure 10:** 3D representation of CIE-L*a*b* color coordinates of silica fabric modified with [$N_{3222}$]Cl ([Si][$N_{3222}$]Cl) regarding aqueous solution of different L-asparagine concentration levels ($10^{-1}$ M, $10^{-2}$ M, $10^{-3}$ M, $10^{-4}$ M and $10^{-5}$ M).

**Figure 11:** 3D representation of CIE-L*a*b* color coordinates of silica fabric modified with [$N_{3114}$]Cl ([Si][$N_{3114}$]Cl) regarding commercial blood human serum with different L-asparagine concentration levels ($10^{-1}$ M, $10^{-2}$ M, $10^{-3}$ M, $10^{-4}$ M and $10^{-5}$ M).

**Figure 12:** 3D representation of CIE-L*a*b* color coordinates of silica fabric modified with [$N_{3222}$]Cl ([Si][$N_{3222}$]Cl) regarding commercial blood human serum with different L-asparagine concentration levels ($10^{-1}$ M, $10^{-2}$ M, $10^{-3}$ M, $10^{-4}$ M and $10^{-5}$ M).

## DETAILED DESCRIPTION

The invention relates to a fabric for detecting a L-asparagine amino acid in a sample as defined in claim 1, wherein the fabric comprises a plurality of silica fibers; an ionic liquid linked to the plurality of silica fibers by a spacer arm, wherein the ionic liquid comprises a quaternary ammonium cation and chloride as counterion; and a L-asparaginase enzyme immobilized in the plurality of silica fibers through the ionic liquid; wherein the presence of L-asparagine in the sample is detectable by a change of color of the fabric after addition of a revealing solution. The use of said fabric as a sensor for detecting L-asparagine amino acid in an aqueous solution, a portable sensor for detecting L-asparagine amino acid in an aqueous solution comprising said fabric, the method for detecting L-asparagine amino acid using said sensor, and a kit comprising said fabric or sensor are also disclosed. Namely, the use of the fabric of the present disclosure for monitoring/diagnosing a disease that causes an increase in the concentration of L-asparagine amino acid in blood, in particular leukemia.

The present disclosure also relates to a fabric for immobilization of L-asparaginase enzyme, the fabric comprising a plurality of silica fibers; an ionic liquid supported in the silica fibers, wherein the ionic liquid comprises a quaternary ammonium cation and chloride as counterion; and wherein the L-asparaginase enzyme is immobilized on the plurality of silica fibers through the ionic liquid. The use of said fabric as a sensor for detecting L-asparagine amino acid in an aqueous solution, a portable sensor for detecting L-asparagine amino acid in an aqueous solution comprising said fabric, the method for detecting L-asparagine amino acid using said sensor, and a kit comprising said fabric or sensor are also disclosed.

The present disclosure relates to a colorimetric sensor for the visual detection of L-asparagine concentration ranges in aqueous solutions. The disclosed biosensor is based on a simple method that, by changing the color of the

material specifically modified to attach the constituent responsible for the quantification of L-asparagine, allows the visual detection of high concentration ranges of L-asparagine in aqueous solutions. In an embodiment, the disclosed sensor can be used in the detection of L-asparagine in the pharmaceutical sector, industrial cooked starched based-foods and in chemical laboratories.

The aim of the present disclosure is to determine by color change the concentration levels of L-asparagine using a portable enzymatic biosensor made with silica fabric functionalized with ionic liquids to attach the L-asparaginase.

In an embodiment, the disclosed biosensor can be used in the pharmaceutical sector, chemical and food industries which need to determine the concentration levels of L-asparagine in aqueous solutions.

**Functionalization of silica fabric with ionic liquids**

In an embodiment, silica fabric was functionalized with quaternary ammonium cations in addition to chloride as the counterion, resulting in a silica fabric functionalized with different functional groups. In particular, the silica fabric was functionalized with a quaternary ammonium cation selected from N,N-dimethylbutylammonium ($[N_{3114}]^+$) and triethylammonium ($[N_{3222}]^+$), in addition to chloride as the counterion, resulting in a silica fabric functionalized with N,N-dimethylbutylammonium chloride ($[Si][N_{3114}]Cl$) or triethylammonium chloride ($[Si][N_{3222}]Cl$), respectively. The abbreviations and chemical structures of the modified silica fabric are displayed in Table 1.

In an embodiment, the silica fabric functionalization was performed through two main steps:

(i) Silica fabric was cut in pieces (3 x 2.5 cm) and then deposited inside a round bottom flask (with a reflux condenser). After, 50 mL of toluene and 5 mL of silane compatibilizing agent/anion source (3-Chloropropyl)trimethoxysilane ($C_6H_{15}ClO_3Si$) was added and kept under reflux for 24 h. Subsequently, the resulting silica fabric was washed with solvents in the following order: 20 mL of toluene, 20 mL of ethanol:water 1:1 (v/v), 30 mL of distilled water, and 20 mL of methanol. After washing, the silica fabric was dried in oven at 37 °C for 24 h, resulting in a modified silica fabric with a chloropropyl spacer arm ($[Si][C_3]Cl$).

(ii) The obtained silica fabric (step i) was put in a round bottom flask with 50 mL of toluene and 5 mL of a cation source, namely $[N_{3114}]^+$ or $[N_{3222}]^+$, and refluxed for 24 h. Afterwards, the material was washed with the following solvents sequence: 20 mL of toluene, 20 mL of methanol, 30 mL of distilled water, and 20 mL of methanol, and dried at 37 °C for 24 h.

**Table 1.** Ionic liquid, abbreviation, and chemical structure of $[Si][C_3]Cl$ and all modified silica fabric materials ($[Si][N_{3114}]Cl$ and $[Si][N_{3222}]Cl$).

| Ionic Liquid | Abbreviation | Chemical Structure |
|---|---|---|
| - | $[Si][C_3]Cl$ | 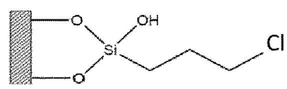 |
| N,N-Dimethylbutylammonium chloride | $[Si][N_{3114}]Cl$ | 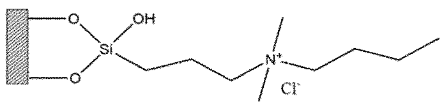 |
| Triethylammonium chloride | $[Si][N_{3222}]Cl$ | 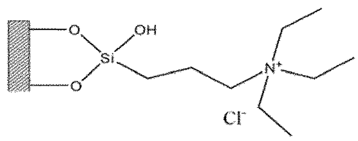 |

Surprisingly, the functionalization with different ionic liquids did not significantly change the color of the functionalized fabric. In an embodiment, the color of the fabric after the functionalization with N,N-dimethylbutylammonium chloride ($[Si][N_{3114}]Cl$) is white-grey (L*:71.861, a*:0.000, and b*:16.413) upon addition of a aqueous solution without L-asparagine and white-grey (L*:64.163, a*:0.466, and b*:21.364) upon the addition of a commercial blood human serum sample without L-asparagine. In another embodiment, the color of the fabric after the functionalization with triethylammonium chloride ($[Si][N_{3222}]Cl$) is white-grey (L*:69.129, a*:1.348, and b*:20.016) upon addition of a aqueous solution without L-asparagine and white-grey (L*: 64.365, a*:2.433, and b*:23.820) upon the addition of a commercial blood human serum sample without L-asparagine.

**Characterization of functionalized silica fabrics**

In an embodiment, all functionalized silica fabrics were characterized by elemental analysis, attenuated total reflectance-Fourier-transform infrared spectroscopy (ATR-FTIR), and scanning electron microscopy (SEM).

In an embodiment, the elemental analysis, i.e., the weight percentages (%w/w) of carbon (%C), hydrogen (%H), and nitrogen (%N) of [Si][$C_3$]Cl and all functionalized silica fabrics, were determined through elemental analysis using the equipment TruSpec 630-200-200, 2 mg of sample, combustion furnace temperature of 1075 °C, and burner temperature of 850 °C. The detection method for carbon and hydrogen was infrared absorption, while for nitrogen was thermal conductivity. The elemental analysis of the modified silica fabric is displayed in Table 2.

**Table 2.** Elemental analysis: weight percentages (%w/w) of carbon (%C), hydrogen (%H), nitrogen (%N), molar amount (n (mol)) used in the synthesis and mass of ionic liquid (IL) per mass of material (i.e., fabric) (mg.g$^{-1}$), of [Si][$C_3$]Cl and all modified silica fabric materials ([Si][$N_{3114}$]Cl and [Si][$N_{3222}$]Cl).

| Material | %C | %H | %N | n (mol) | IL mass per material mass (mg.g$^{-1}$) |
|---|---|---|---|---|---|
| Silica fabric | 0.824 | 1.075 | 0.137 | - | - |
| [Si][$C_3$]Cl | 3.701 | 1.593 | 0.119 | - | - |
| [Si][$N_{3114}$]Cl | 2.993 | 1.189 | 0.333 | 0.035 | 25.009 |
| [Si[$N_{3222}$]Cl | 2.401 | 1.035 | 0.203 | 0.036 | 8.421 |

In an embodiment, the mass of IL per mass of material (mg.g$^{-1}$) was obtained using the following formula (M - molar mass (g.mol$^{-1}$); %N - weight percentage of nitrogen):

$$\left(\frac{\frac{(\%N_{modified\ silica\ fabric} - \%N_{silica\ fabric\ material}) \times M_{ionic\ liquid}}{N_{mass\ per\ ionic\ liquid}}}{100}\right) \times 1000$$

In an embodiment, spectra from attenuated total reflectance - Fourier-transform infrared (ATR-FTIR) spectroscopy was obtained using Perkin Elmer FT-IR System Spectrum BX equipment (Waltham, MA, USA) and a solid sample of all functionalized silica fabrics at 25 °C and in a range between 4000-500 cm$^{-1}$. Each sample was scanned 64 times with a resolution of 8.0 cm$^{-1}$.

In an embodiment, scanning electron microscopy (SEM) was performed using a Hitachi SU-70 microscope at the accelerating voltage of 15 kV. A carbon thin film deposition was used in order to increase the samples' conductivity. Fiber diameter of silica fabric and all functionalized silica fabrics were defined using the line tool in ImageJ (National Institute of Mental Health, Bethesda, Maryland, USA). The average fiber diameter of the modified silica fabric is displayed in Table 3.

**Table 3.** Average fiber diameter of silica fabric and all modified silica fabric materials ([Si][$N_{3114}$]Cl and [Si][$N_{3222}$]Cl).

| Material | Average fiber diameter (μm) |
|---|---|
| Silica fabric | 8.38±0.76 |
| [Si][$N_{3114}$]Cl | 8.53±1.39 |
| [Si][$N_{3222}$]Cl | 8.40±0.94 |

In an embodiment, the colorimetric biosensors were developed through two main steps: (i) L-Asparaginase physical adsorption on functionalized silica fabrics and (ii) revealing solution addition. In the first step, the enzyme L-asparaginase was immobilized on functionalized silica fabrics at room temperature through the addition of 400 μL of L-asparaginase solution (0.6 mg mL$^{-1}$) into 1 cm x 1 cm of functionalized silica fabrics: [Si][$N_{3114}$]Cl and [Si][$N_{3222}$]Cl. Subsequently, 400 μL of aqueous samples (solution of different L-asparagine concentration levels, $10^{-1}$ M, $10^{-2}$ M, $10^{-3}$ M, $10^{-4}$ M and $10^{-5}$ M, or commercial blood human serum with the same L-asparagine concentration levels) was added to the functionalized silica fabrics. Then, 400 μL of revealing solution, Nessler's reagent, is added. After 20 seconds, the resulting color of the functionalized silica fabrics, was dependent on the L-asparagine concentration levels of each sample.

In an embodiment, the resulting colors of functionalized silica fabrics were analyzed by a Portable Spectrophotometer CM-2300d (Konica Minolta Europe). Color changes were quantified using the color organization system, i.e., CIELAB (or CIE L* a* b*) color space, which expresses the color's lightness, red/green and yellow/blue intensity, as L*, a*,

and b* values, respectively. These chromaticity coordinates were converted into RGB (Red, Green, Blue) color system using the conversion spreadsheets (available from http://colormine.org/convert/rgb-to-lab (accessed on 17 February 2022)).

## Examples of application

The potential applications of the present biosensor for the quantification ranges of L-asparagine are in the pharmaceutical sector, food and chemical industries.

In an embodiment, in the pharmaceutical sector the biosensor can be used to determine concentration levels of L-asparagine in blood serum samples. The determined concentration of L-asparagine levels can be used to monitor the treatment response in disease therapy with L-asparaginase. L-asparagine concentration in leukemia blood serum samples is higher than detected in healthy blood serum samples. More specifically, the concentration of L-asparagine in leukemia blood serum samples varies from $10^{-3}$ to $10^{-2}$ M, while in healthy blood serum samples varies from $10^{-6}$ to $10^{-4}$ M [1-6]. In an embodiment, in food industries, starch-rich foods cooked at temperatures over 100 °C led to the formation of a carcinogenic compound, acrylamide, through the Maillard reaction between L-asparagine and carbonyl compounds. Thus, the present biosensor can be used to detect and quantify the L-asparagine levels in these foods, from a liquid food-derived sample, to avoid the formation of high levels of acrylamide (for example acrylamide carcinogenic levels).

In an embodiment, in chemical industries, the present biosensor can be applied in any chemical process that needs the detection of L-asparagine levels.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above-described embodiments are combinable.

The following claims set out present invention.

References

[1] N. Verma, K. Kumar, G. Kaur, S. Anand, E. coli K-12 asparaginase-based asparagine biosensor for leukemia, Artif. Cells, Blood Substitutes, Biotechnol. 35 (2007) 449-456. https://doi.org/10.1080/10731190701460358.

[2] K. Kumar, S. Walia, L-asparaginase extracted from Capsicum annum L and development of asparagine biosensor for leukemia, Sens. Transducers. 144 (2012) 192-200.

[3] K. Kumar, S. Punia, J. Kaur, T. Pathak, Development of plant asparagine biosensor for detection of leukemia, J Pharm Biomed Sci. 35 (2013) 1796-1801.

[4] K. Kumar, M. Kataria, N. Verma, Plant asparaginase-based asparagine biosensor for leukemia, Artif Cells Nanomed Biotechnol. 41 (2013) 184-188. https://doi.org/10.3109/10731199.2012.716062.

[5] T. Pathak, R. Kumar, J. Kaur, K. Kumar, Isolation of L-asparaginase from Cannabis Sativa and development of biosensor for detection of asparagine in leukemic serum samples, Res. J. Pharm. Technol. 7 (2014) 850-855.

[6] S. Punia, R. Kumar, K. Kumar, Enzyme based asparagine biosensor for the detection of asparagine levels in leukemic samples, Int. J. Appl. Biol. Pharm. Technol. 6 (2015) 40-43.


## Claims

**1.** A fabric for detecting a L-asparagine amino acid in a sample, wherein the fabric comprises:

a plurality of silica fibers;
an ionic liquid linked to the plurality of silica fibers by a spacer arm, wherein the ionic liquid comprises a quaternary ammonium cation and a chloride as counterion; and
a L-asparaginase enzyme immobilized in the plurality of silica fibers through the ionic liquid;
wherein the spacer arm comprises a compound with formula $C_xH_yClO_3Si$, selected from a list comprising (3-Chloropropyl)trimethoxysilane ($C_6H_{15}ClO_3Si$), (3-Chloropropyl)triethoxysilane ($C_9H_{21}ClO_3Si$), (Chloromethyl) trimethoxysilane ($C_4H_{11}ClO_3Si$), (Chloromethyl)triethoxysilane ($C_7H_{17}ClO_3Si$), (2-Chloroethyl)trimethoxysilane ($C_5H_{13}ClO_3Si$), (2-Chloroethyl)triethoxysilane ($C_8H_{19}ClO_3Si$), (4-Chlorobutyl)trimethoxysilane ($C_7H_{17}ClO_3Si$), (4-Chlorobutyl)triethoxysilane ($C_{10}H_{23}ClO_3Si$), or combinations thereof; and
wherein the fabric is configured to change color in the presence of L-asparagine in the sample after the addition of

a revealing solution.

**2.** The fabric according to the previous claim wherein the spacer arm is a (3-Chloropropyl)trimethoxysilane.

**3.** The fabric according to any of the previous claims wherein the concentration of L-asparaginase enzyme in the fabric ranges from 0.1 mg/cm$^2$ to 0.5 mg/cm$^2$.

**4.** The fabric according to any of the previous claims wherein the silica fibers are woven.

**5.** The fabric according to any of the previous claims wherein the quaternary ammonium cation comprised in the ionic liquid is selected from N,N-dimethylbutylammonium or triethylammonium.

**6.** The fabric according to any of the previous claims wherein the mass of ionic liquid per mass of fabric ranges from 8 mg.g$^{-1}$ to 30 mg.g$^{-1}$, preferably from 8 mg.g$^{-1}$ to 25 mg.g$^{-1}$.

**7.** The fabric according to any of the previous claims wherein the diameter of the silica fibers ranges from 7 to 11 $\mu$m, measured by scanning electron microscopy.

**8.** The fabric according to any of the previous claims wherein the sample is a liquid biological sample, or a liquid food-derived sample, preferably wherein the liquid biological sample is a blood serum sample.

**9.** A portable sensor for detecting L-asparagine amino acid in an aqueous solution comprising a fabric as described in any of the previous claims 1-8, preferably a sensor for monitoring and/or diagnosing a disease that causes an increase in the concentration of L-asparagine amino acid in blood.

**10.** The portable sensor according to the previous claim wherein the sensor is arranged to detect L-asparagine amino acid in a concentration ranging from 10$^{-5}$ M to 10 M, preferably 10$^{-3}$ M to 10$^{-1}$ M.

**11.** A method for detecting L-asparagine amino acid in an aqueous solution using a fabric as described in any of previous claims 1-8, or a portable sensor comprising the fabric as described in any of previous claims 9-10, the method comprising the following steps:

adding the aqueous solution to the fabric or portable sensor;
adding a revealing solution to the fabric or portable sensor, preferably wherein the revealing solution is Nessler's reagent;
analyzing the fabric or portable sensor for a change of color, wherein a change of color indicates the presence of L-asparagine amino acid in the aqueous solution.

**12.** Use of the fabric described in any of the previous claims as a sensor for detecting L-asparagine amino acid in an aqueous solution, preferably wherein the aqueous solution is a blood serum sample, or a food-derived sample.

**13.** A kit for detecting L-asparagine amino acid, preferably for monitoring and/or diagnosing a disease that causes an increase in the concentration of L-asparagine amino acid in blood, comprising the fabric as described in any of the previous claims 1-8, or the portable sensor described in any of the previous claims 9-10, and a revealing solution, wherein the revealing solution is Nessler's reagent.

## Patentansprüche

**1.** Ein Gewebe zum Nachweis einer L-Asparagin-Aminosäure in einer Probe, wobei das Gewebe umfasst:

eine Vielzahl von Siliciumdioxid-Fasern;
eine ionische Flüssigkeit, die durch einen Abstandshalter (*spacer arm*) mit der Vielzahl von Siliciumdioxid-Fasern verbunden ist, wobei die ionische Flüssigkeit ein quartäres Ammonium-Kation und ein Chlorid als Gegenion umfasst; und
ein durch die ionische Flüssigkeit in der Vielzahl von Siliciumdioxid-Fasern immobilisiertes L-Asparaginase-Enzym;
wobei der Abstandshalter eine Verbindung mit der Formel $C_xH_yClO_3Si$ umfasst, ausgewählt aus einer Liste, die

(3-Chlorpropyl)trimethoxysilan ($C_6H_{15}Clo_3Si$), (3-Chlorpropyl)triethoxysilan ($C_9H_{21}ClO_3Si$), (Chlormethyl)trimethoxysilan ($C_4H_{11}ClO_3Si$), (Chlormethyl)triethoxysilan ($C_7H_{17}ClO_3Si$), (2-Chlorethyl)trimethoxysilan ($C_5H_{13}ClO_3Si$), (2-Chlorethyl)triethoxysilan ($C_8H_{19}ClO_3Si$), (4-Chlorbutyl)trimethoxysilan ($C_7H_{17}ClO_3Si$), (4-Chlorbutyl)triethoxysilan ($C_{10}H_{23}ClO_3Si$), oder Kombinationen davon umfasst; und
wobei das Gewebe so konfiguriert ist, dass es in Gegenwart von L-Asparagin in der Probe nach Zugabe einer Nachweismittel die Farbe ändert.

**2.** Das Gewebe nach dem vorangehenden Anspruch, wobei der Abstandhalter (3-Chlorpropyl)trimethoxysilan ist.

**3.** Das Gewebe nach einem der vorangehenden Ansprüche, wobei die Konzentration des L-Asparaginase-Enzyms in dem Gewebe im Bereich von 0,1 $mg/cm^2$ bis 0,5 $mg/cm^2$ liegt.

**4.** Das Gewebe nach einem der vorangehenden Ansprüche, wobei die Siliciumdioxid-Fasern gewebt sind.

**5.** Das Gewebe nach einem der vorangehenden Ansprüche, wobei das in der ionischen Flüssigkeit enthaltene quartäre Ammonium-Kation ausgewählt ist aus N,N-Dimethylbutylammonium oder Triethylammonium.

**6.** Das Gewebe nach einem der vorangehenden Ansprüche, wobei die Masse der ionischen Flüssigkeit pro Gewebemasse im Bereich von 8 $mg.g^{-1}$ bis 30 $mg.g^{-1}$, bevorzugt von 8 $mg.g^{-1}$ bis 25 $mg.g^{-1}$, liegt.

**7.** Das Gewebe nach einem der vorangehenden Ansprüche, wobei der Durchmesser der Siliciumdioxid-Fasern im Bereich von 7 bis 11 $\mu$m liegt, gemessen per Rasterelektronenmikroskopie.

**8.** Das Gewebe nach einem der vorangehenden Ansprüche, wobei die Probe eine flüssige biologische Probe oder eine von einem Lebensmittel stammende flüssige Probe ist, wobei die flüssige biologische Probe bevorzugt eine Probe eines Blutserums ist.

**9.** Ein tragbarer Sensor zum Nachweis von L-Asparagin-Aminosäure in einer wässrigen Lösung, umfassend ein Gewebe, wie in einem der vorangehenden Ansprüche 1-8 beschrieben, bevorzugt ein Sensor zur Überwachung und/oder Diagnose einer Krankheit, die einen Anstieg der Konzentration von L-Asparagin-Aminosäure im Blut verursacht.

**10.** Der tragbare Sensor nach dem vorangehenden Anspruch, wobei der Sensor so angeordnet ist, dass er L-Asparagin-Aminosäure in einer Konzentration im Bereich von $10^{-5}$ M bis 10 M, bevorzugt $10^{-3}$ M bis $10^{-1}$ M, nachweist.

**11.** Ein Verfahren zum Nachweis von L-Asparagin-Aminosäure in einer wässrigen Lösung unter Verwendung eines Gewebes, wie in einem der vorangehenden Ansprüche 1-8 beschrieben, oder eines tragbaren Sensors, umfassend das Gewebe, wie in einem der vorangehenden Ansprüche 9-10 beschrieben, wobei das Verfahren die folgenden Schritte umfasst:

Hinzufügen der wässrigen Lösung zu dem Gewebe oder dem tragbaren Sensor;
Hinzufügen eines Nachweismittels zu dem Gewebe oder dem tragbaren Sensor, wobei das Nachweismittel bevorzugt ein Neßler-Reagenz ist;
Analysieren des Gewebes oder des tragbaren Sensors auf eine Farbveränderung, wobei eine Farbveränderung das Vorhandensein von L-Asparagin-Aminosäure in der wässrigen Lösung anzeigt.

**12.** Verwenden des in einem der vorangehenden Ansprüche beschriebenen Gewebes als ein Sensor zum Nachweis von L-Asparagin-Aminosäure in einer wässrigen Lösung, wobei es sich bei der wässrigen Lösung bevorzugt um eine Probe eines Blutserums oder eine von einem Lebensmittel stammende Probe handelt.

**13.** Ein Kit zum Nachweis von L-Asparagin-Aminosäure, bevorzugt zur Überwachung und/oder Diagnose einer Krankheit, die einen Anstieg der Konzentration von L-Asparagin-Aminosäure im Blut verursacht, umfassend das in einem der vorangehenden Ansprüche 1-8 beschriebene Gewebe oder den in einem der vorangehenden Ansprüche 9-10 beschriebenen tragbaren Sensor und eine Nachweismittel, wobei die Nachweismittel ein Neßler-Reagenz ist.

## Revendications

1. Un tissu pour la détection d'un acide aminé L-asparagine dans un échantillon, dans lequel le tissu comprend :

   une pluralité de fibres de silice ;
   un liquide ionique lié à la pluralité de fibres de silice par un bras espaceur, dans lequel le liquide ionique comprend un cation ammonium quaternaire et un chlorure en tant que contre-ion ; et
   une enzyme L-asparaginase immobilisée dans la pluralité de fibres de silice à travers le liquide ionique ;
   dans lequel le bras espaceur comprend un composé de formule $C_xH_yClO_3Si$, choisi parmi une liste comprenant du (3-Chloropropyl)triméthoxysilane ($C_6H_{15}ClO_3Si$), (3-Chloropropyl)triéthoxysilane ($C_9H_{21}ClO_3Si$), (Chlorométhyl)triméthoxysilane ($C_4H_{11}ClO_3Si$), (Chlorométhyl)triéthoxysilane ($C_7H_{17}ClO_3Si$), (2-Chloroéthyl)triméthoxysilane ($C_5H_{13}ClO_3Si$), (2-Chloroéthyl)triéthoxysilane ($C_8H_{19}ClO_3Si$), (4-Chlorobutyl)triméthoxysilane ($C_7H_{17}ClO_3Si$), (4-Chlorobutyl)triéthoxysilane ($C_{10}H_{23}ClO_3Si$), ou des combinaisons de ceux-ci ; et
   dans lequel le tissu est configuré pour changer de couleur en présence de L-asparagine dans l'échantillon après l'ajout d'une solution révélatrice.

2. Le tissu selon la revendication précédente dans lequel le bras espaceur est un (3-Chloropropyl)triméthoxysilane.

3. Le tissu selon l'une quelconque des revendications précédentes dans lequel la concentration de l'enzyme L-asparaginase dans le tissu varie de 0,1 $mg/cm^2$ à 0,5 $mg/cm^2$.

4. Le tissu selon l'une quelconque des revendications précédentes, dans lequel les fibres de silice sont tissées.

5. Le tissu selon l'une quelconque des revendications précédentes dans lequel le cation ammonium quaternaire compris dans le liquide ionique est choisi parmi du N,N-diméthylbutylammonium ou triéthylammonium.

6. Le tissu selon l'une quelconque des revendications précédentes dans lequel la masse de liquide ionique par masse de tissu varie de 8 $mg.g^{-1}$ à 30 $mg.g^{-1}$, préférablement de 8 $mg.g^{-1}$ à 25 $mg.g^{-1}$.

7. Le tissu selon l'une quelconque des revendications précédentes dans lequel le diamètre des fibres de silice varie de 7 à 11 $\mu$m, mesuré par microscopie électronique à balayage.

8. Le tissu selon l'une quelconque des revendications précédentes dans lequel l'échantillon est un échantillon biologique liquide, ou un échantillon dérivé d'aliments liquide, préférablement dans lequel l'échantillon biologique liquide est un échantillon de sérum sanguin.

9. Un capteur portable pour la détection de l'acide aminé L-asparagine dans une solution aqueuse comprenant un tissu tel que décrit dans l'une quelconque des revendications précédentes 1-8, préférablement un capteur pour faire le suivi et/ou diagnostiquer une maladie qui cause une augmentation dans la concentration d'acide aminé L-asparagine dans le sang.

10. Le capteur portable selon la revendication précédente dans lequel le capteur est arrangé pour détecter l'acide aminé L-asparagine à une concentration variant de $10^{-5}$ M à 10 M, préférablement de $10^{-3}$ M à $10^{-1}$ M.

11. Un procédé pour la détection de l'acide aminé L-asparagine dans une solution aqueuse à l'aide d'un tissu tel que décrit dans l'une quelconque des revendications précédentes 1-8, ou un capteur portable comprenant le tissu tel que décrit dans l'une quelconque des revendications précédentes 9-10, le procédé comprenant les étapes suivantes :

    ajouter la solution aqueuse au tissu ou capteur portable ;
    ajouter une solution révélatrice au tissu ou capteur portable, préférablement dans laquelle la solution révélatrice est le réactif de Nessler ;
    analyser le tissu ou le capteur portable en vue d'un changement de couleur, dans lequel un changement de couleur indique la présence de l'acide aminé L-asparagine dans la solution aqueuse.

12. Utilisation du tissu décrit dans l'une quelconque des revendications précédentes en tant que capteur pour détecter l'acide aminé L-asparagine dans une solution aqueuse, préférablement dans lequel la solution aqueuse est un échantillon de sérum sanguin, ou un échantillon dérivé d'aliments.

**13.** Un kit pour détecter l'acide aminé L-asparagine, préférablement pour surveiller et/ou diagnostiquer une maladie qui cause une augmentation dans la concentration de l'acide aminé L-asparagine dans le sang, comprenant le tissu tel que décrit dans l'une quelconque des revendications précédentes 1-8, ou le capteur portable décrit dans l'une quelconque des revendications précédentes 9-10, et une solution révélatrice, dans lequel la solution révélatrice est le réactif de Nessler.

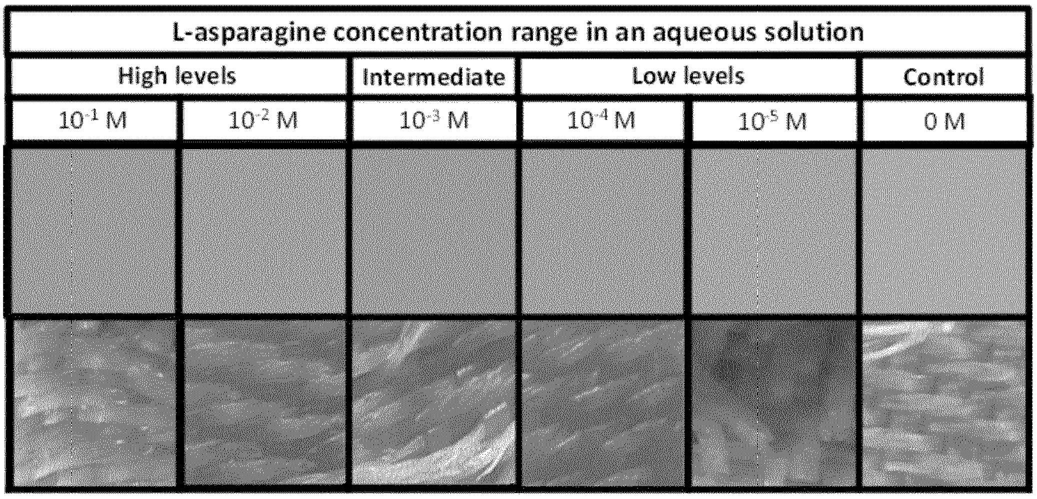
L-asparagine concentration range in an aqueous solution
High levels
Intermediate
Low levels
Control
$10^{-1}$ M
$10^{-2}$ M
$10^{-3}$ M
$10^{-4}$ M
$10^{-5}$ M
0 M

**Fig. 1**

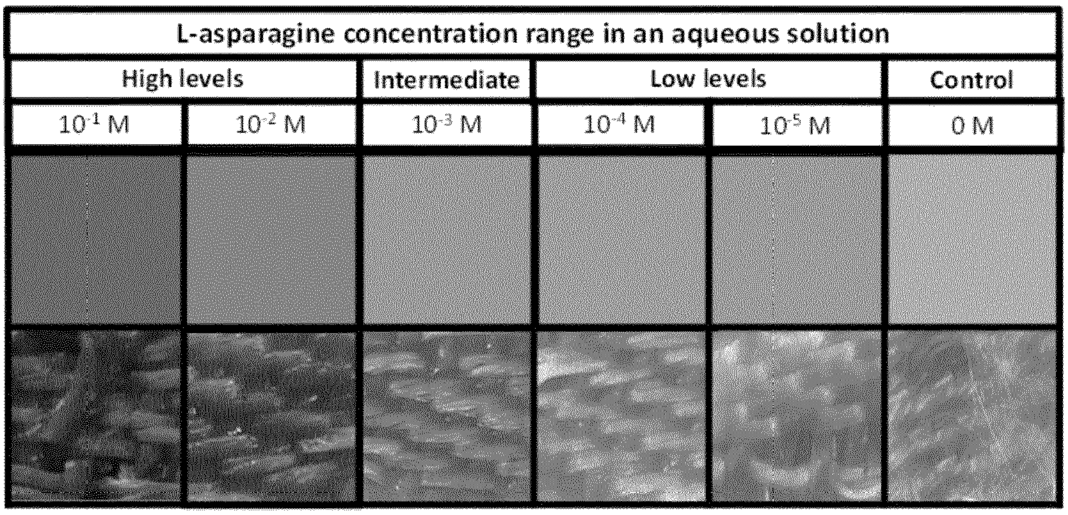
L-asparagine concentration range in an aqueous solution
High levels
Intermediate
Low levels
Control
$10^{-1}$ M
$10^{-2}$ M
$10^{-3}$ M
$10^{-4}$ M
$10^{-5}$ M
0 M

**Fig. 2**

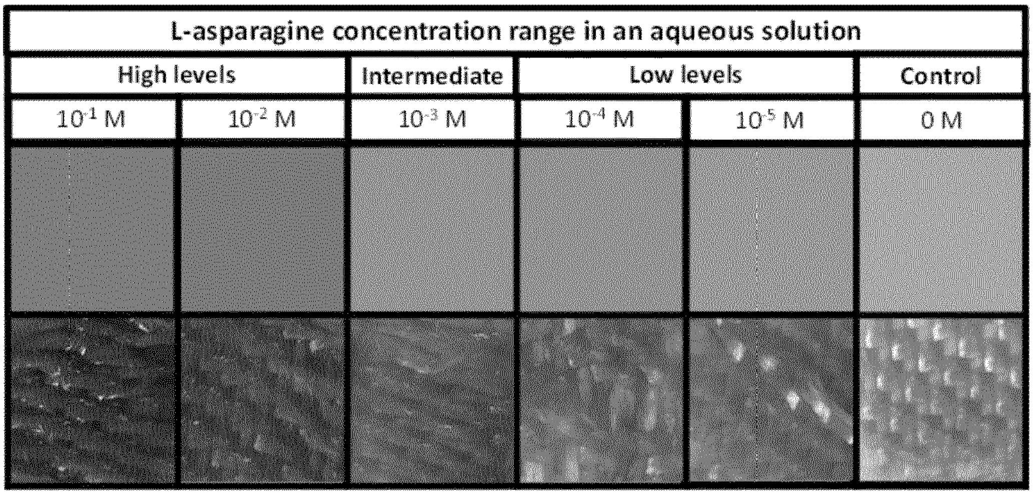
L-asparagine concentration range in an aqueous solution
High levels
Intermediate
Low levels
Control
$10^{-1}$ M
$10^{-2}$ M
$10^{-3}$ M
$10^{-4}$ M
$10^{-5}$ M
0 M

**Fig. 3**

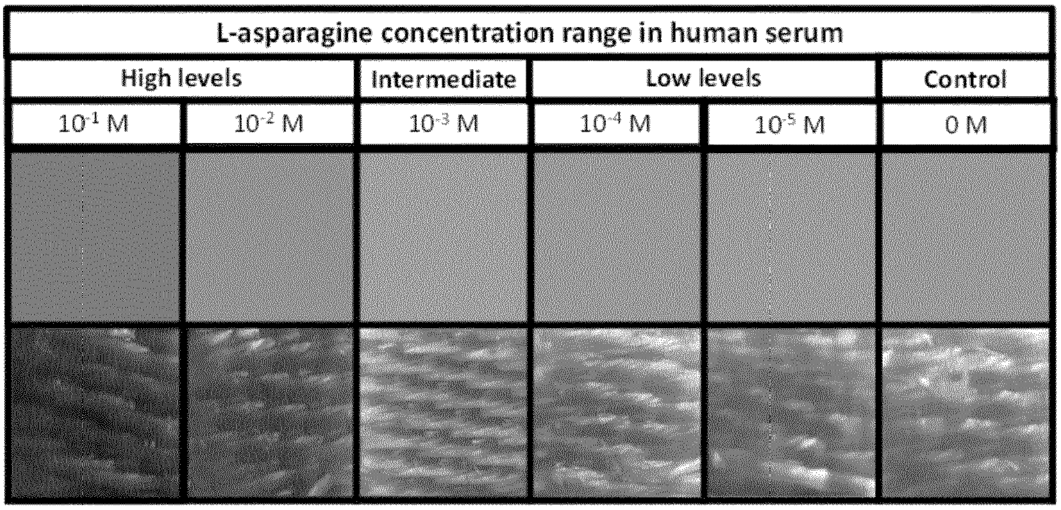
L-asparagine concentration range in human serum
High levels
Intermediate
Low levels
Control
$10^{-1}$ M
$10^{-2}$ M
$10^{-3}$ M
$10^{-4}$ M
$10^{-5}$ M
0 M

**Fig. 4**

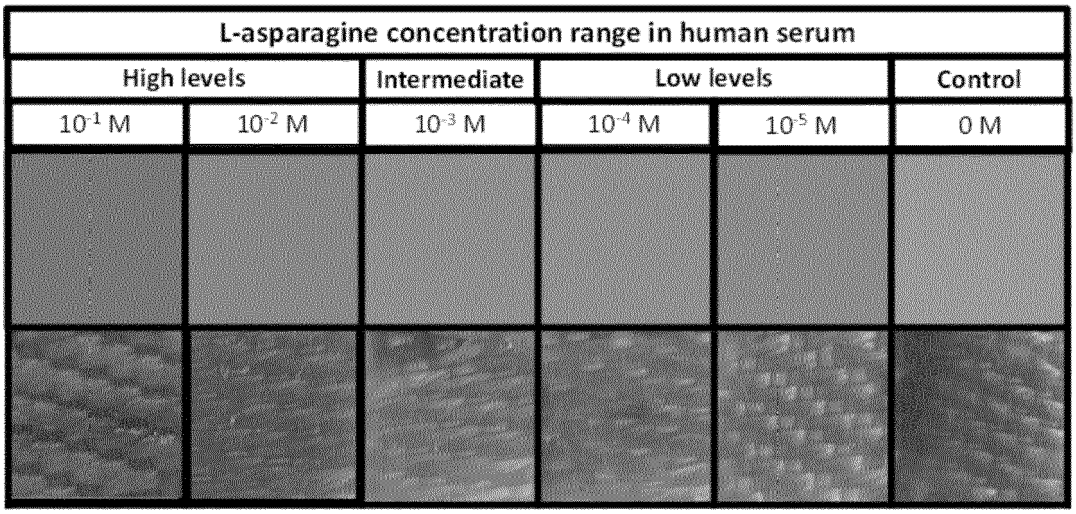
L-asparagine concentration range in human serum
High levels
Intermediate
Low levels
Control
$10^{-1}$ M
$10^{-2}$ M
$10^{-3}$ M
$10^{-4}$ M
$10^{-5}$ M
0 M

**Fig. 5**

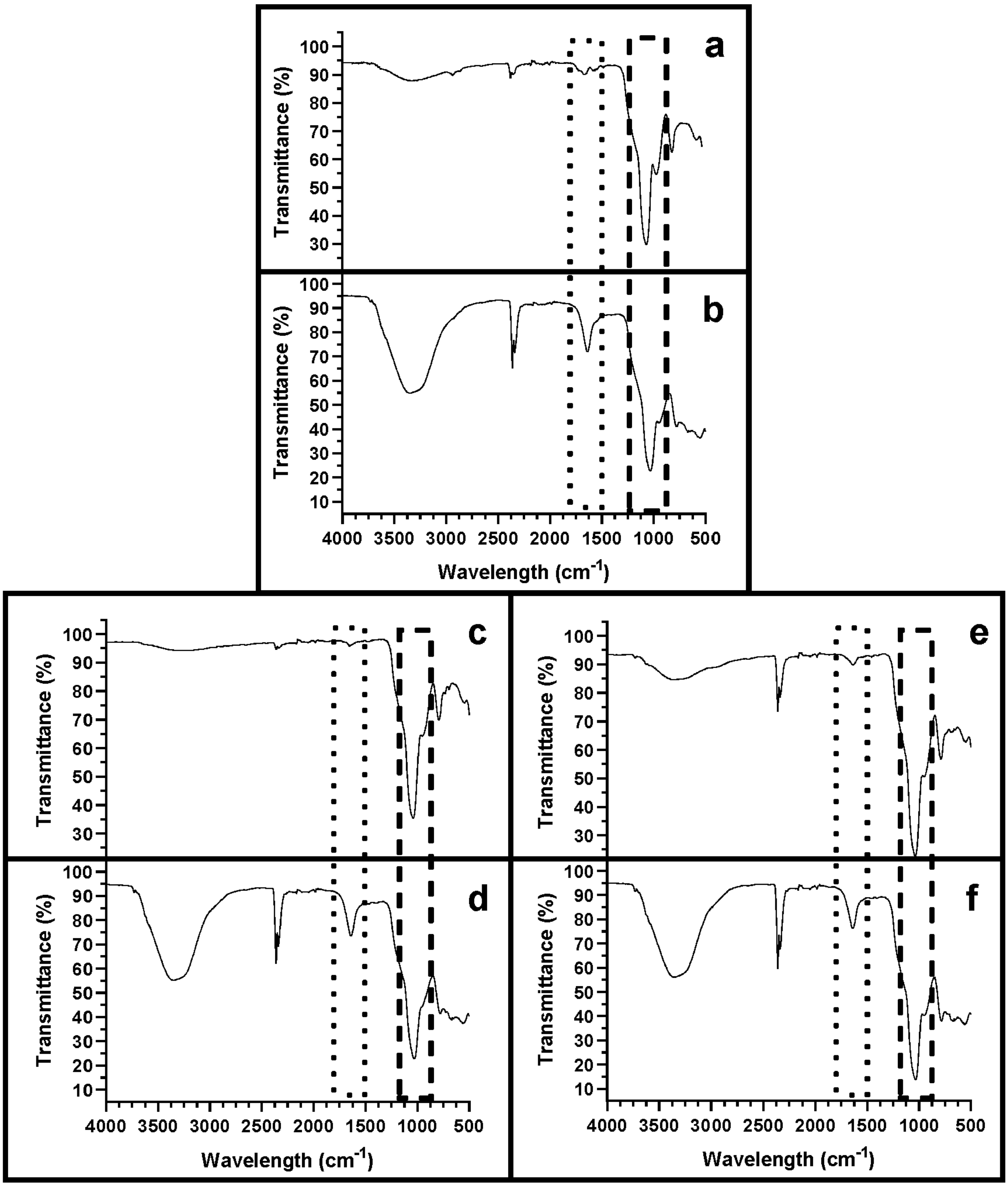

a
b
c
d
e
f
Transmittance (%)
Wavelength (cm⁻¹)
4000 3500 3000 2500 2000 1500 1000 500


Fig. 6

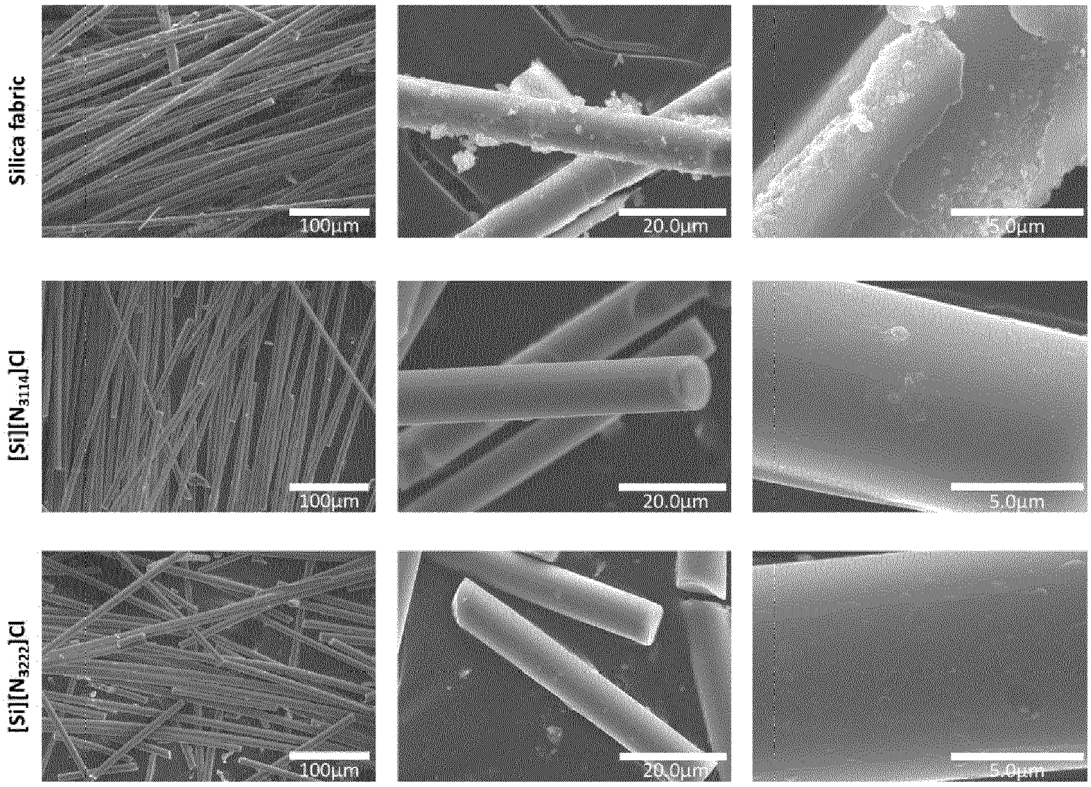


Fig. 7

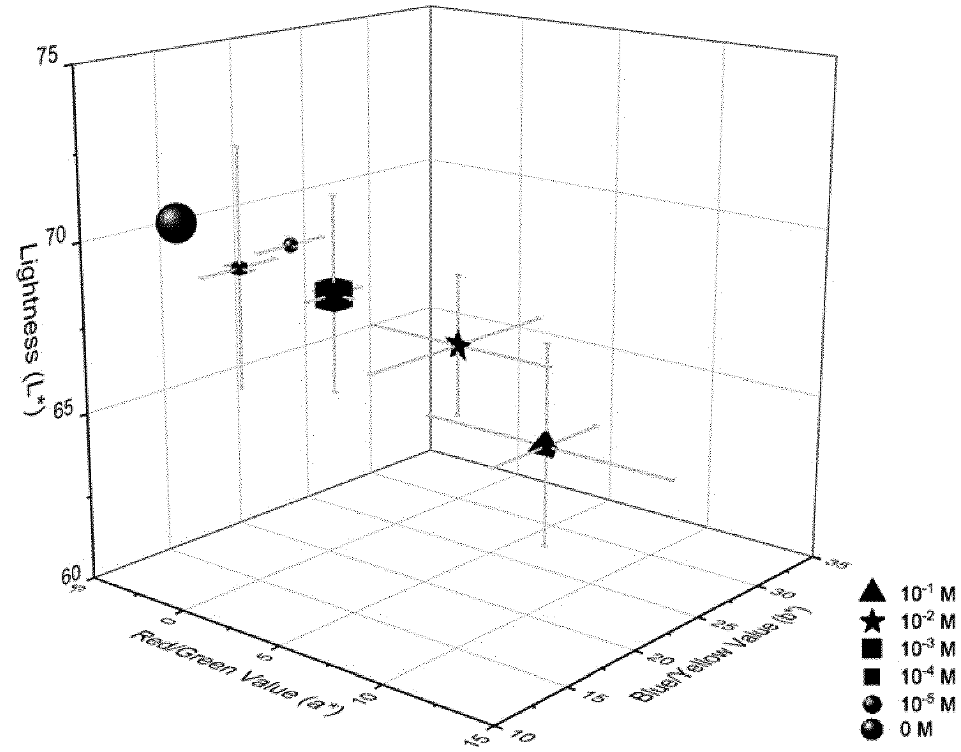
Lightness (L*)
Red/Green Value (a*)
Blue/Yellow Value (b*)
$10^{-1}$ M
$10^{-2}$ M
$10^{-3}$ M
$10^{-4}$ M
$10^{-5}$ M
0 M

Fig. 8

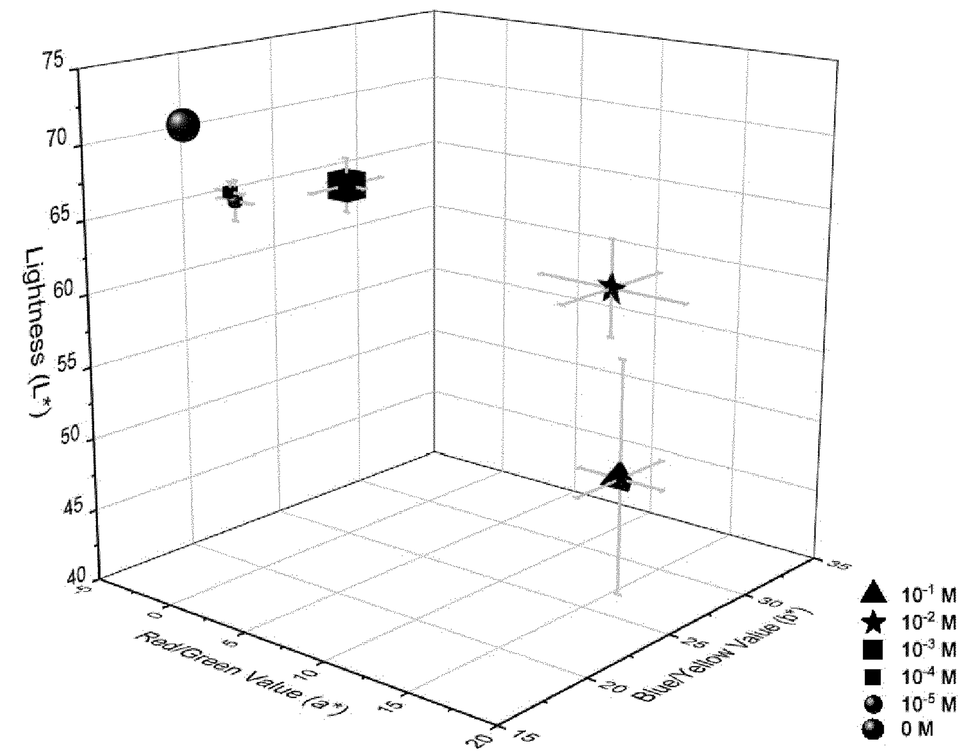
Lightness (L*)
Red/Green Value (a*)
Blue/Yellow Value (b*)
$10^{-1}$ M
$10^{-2}$ M
$10^{-3}$ M
$10^{-4}$ M
$10^{-5}$ M
0 M

Fig. 9

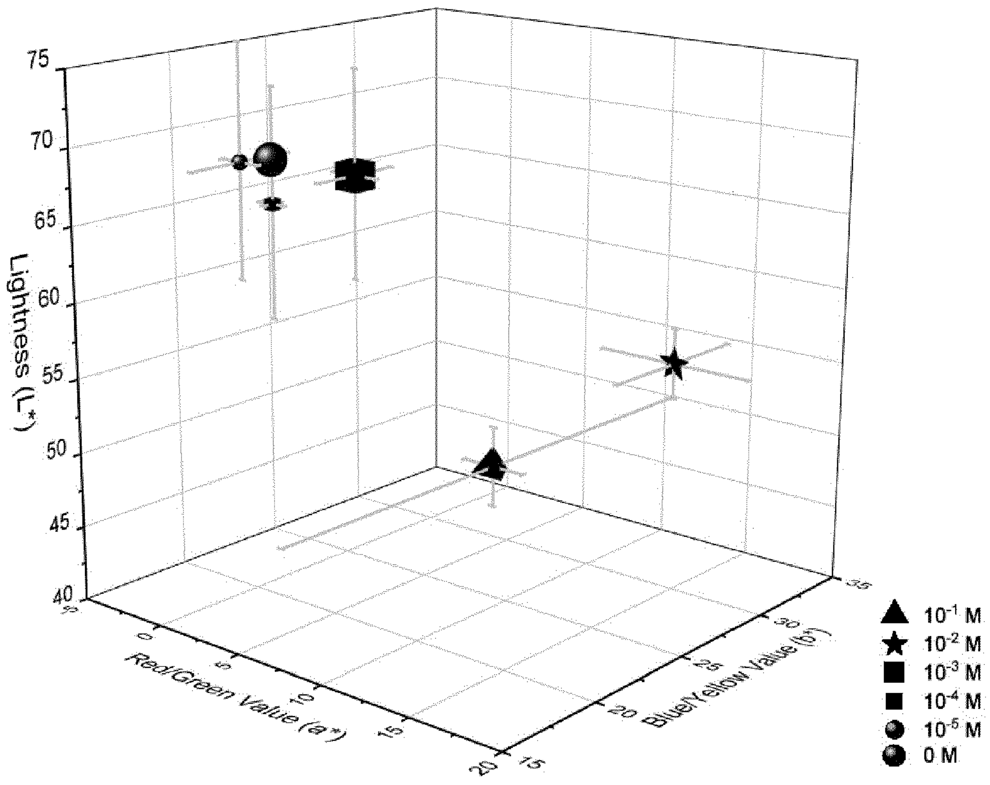
Lightness (L*)
Red/Green Value (a*)
Blue/Yellow Value (b*)
10-1 M
10-2 M
10-3 M
10-4 M
10-5 M
0 M

Fig. 10

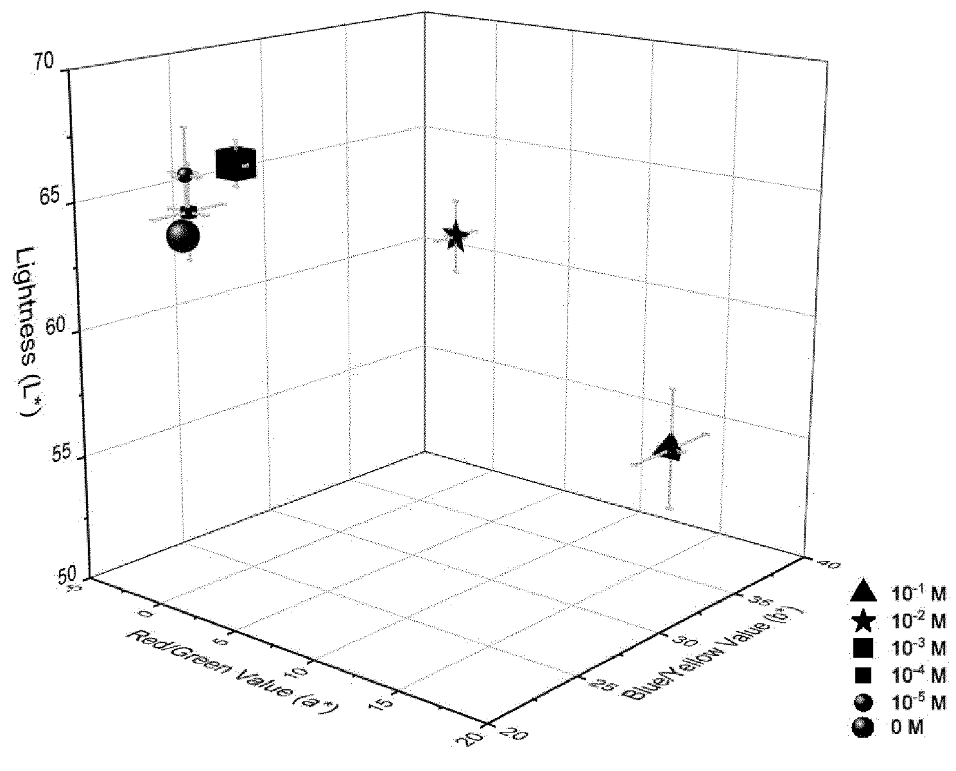
Lightness (L*)
Red/Green Value (a*)
Blue/Yellow Value (b*)
10-1 M
10-2 M
10-3 M
10-4 M
10-5 M
0 M

Fig. 11

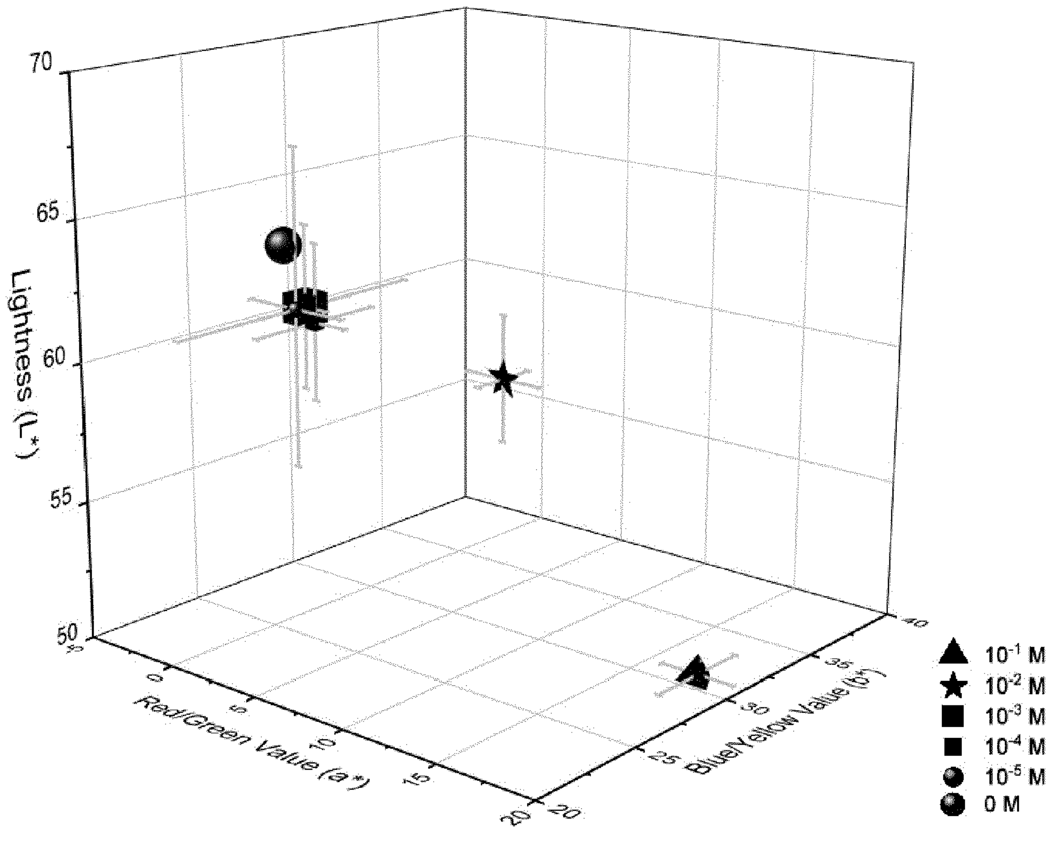
Lightness (L*)
70
65
60
55
50
Red/Green Value (a*)
0
5
10
15
20
Blue/Yellow Value (b*)
20
25
30
35
40
10⁻¹ M
10⁻² M
10⁻³ M
10⁻⁴ M
10⁻⁵ M
0 M

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20100190658 A1
- WO 199405805 A1
- WO 2008013530 A1


### Non-patent literature cited in the description


- L-Asparaginase-based biosensors. **NUNES, J.C.F.** ; **CRISTÓVÃO, R.O.** ; **SANTOS-EBINUMA, V.C** ; **FARIA, J.L.** ; **SILVA, C.G.** ; **NEVES, M.C.** ; **FREIRE, M.G.** ; **TAVARES, A.P.M.** Encyclopedia. 2021, vol. 1, 848-858
- **NUNES, J.C.F** ; **ALMEIDA, M.R.** ; **BENTO, R.M.F.** ; **PEREIRA, M.M.** ; **SANTOS-EBINUMA, V.C.** ; **NEVES, M.C.** ; **FREIRE, M.G** ; **TAVARES, A.P.M.** Enhanced enzyme reuse through the bioconjugation of L-asparaginase and silica-based supported ionic liquid-like phase materials.. *Molecules.*, 2022, vol. 27, 1-21
- **KOTZIA, G.A.** ; **LABROU, N.E.** Engineering substrate specificity of E. carotovora L-asparaginase for the development of biosensor.. *J. Mol. Catal. B Enzym.*, 2011, vol. 72, 95-101
- **LABROU, N.E.** ; **MUHARRAM, M.M.** Biochemical characterization and immobilization of Erwinia carotovora L-asparaginase in a microplate for high-throughput biosensing of L-asparagine.. *Enzym. Microb. Technol*, 2016, vol. 92, 86-93
- **N. VERMA** ; **K. KUMAR** ; **G. KAUR** ; **S. ANAND**. E. coli K-12 asparaginase-based asparagine biosensor for leukemia. *Artif. Cells, Blood Substitutes, Biotechnol.*, 2007, vol. 35, 449-456, https://doi.org/10.1080/10731190701460358
- **K. KUMAR** ; **S. WALIA**. L-asparaginase extracted from Capsicum annum L and development of asparagine biosensor for leukemia. *Sens. Transducers.*, 2012, vol. 144, 192-200
- **K. KUMAR** ; **S. PUNIA** ; **J. KAUR** ; **T. PATHAK**. Development of plant asparagine biosensor for detection of leukemia. *J Pharm Biomed Sci.*, 2013, vol. 35, 1796-1801
- **K. KUMAR** ; **M. KATARIA** ; **N. VERMA**. Plant asparaginase-based asparagine biosensor for leukemia. *Artif Cells Nanomed Biotechnol*, 2013, vol. 41, 184-188, https://doi.org/10.3109/10731199.2012.716062
- **T. PATHAK** ; **R. KUMAR** ; **J. KAUR** ; **K. KUMAR**. Isolation of L-asparaginase from Cannabis Sativa and development of biosensor for detection of asparagine in leukemic serum samples. *Res. J. Pharm. Technol.*, 2014, vol. 7, 850-855
- **S. PUNIA** ; **R. KUMAR** ; **K. KUMAR**. Enzyme based asparagine biosensor for the detection of asparagine levels in leukemic samples. *Int. J. Appl. Biol. Pharm. Technol*, 2015, vol. 6, 40-43